(19) Europäisches Patentamt
European Patent Office
Office européen des brevets

(11) Veröffentlichungsnummer: **0 379 162 A2**

(12) **EUROPÄISCHE PATENTANMELDUNG**

(21) Anmeldenummer: **90100890.4**

(51) Int. Cl.⁵: **C07K 7/40**

(22) Anmeldetag: **17.01.90**

(30) Priorität: **21.01.89 DE 3901718**

(43) Veröffentlichungstag der Anmeldung: **25.07.90 Patentblatt 90/30**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Dörschug, Michael, Dr.**
**Sonnenleite 20**
**D-4630 Bochum 7(DE)**

(54) **Verfahren zur Renaturierung falscher Rekombinanten von Insulinvorläufern.**

(57) "Falsche" Rekombinanten von Insulin-Vorläufern werden in wäßrigem Medium mit überschüssigem Mercaptan in Gegenwart eines organischen Redox-Systems oder mindestens einer organischen Verbindung, welche unter den Reaktionsbedingungen ein solches organisches Redox-System bildet, renaturiert, d.h. in die "richtigen" Rekombinanten in einem einzigen Reaktionsschritt umgewandelt.

Die "richtigen" Rekombinanten können enzymatisch oder chemisch nach bekannten Techniken in das entsprechende Insulin überführt werden.

EP 0 379 162 A2

## Verfahren zur Renaturierung falscher Rekombinanten von Insulinvorläufern

Insulin ist ein Molekül, das aus 2 Polypeptidketten besteht, die über Disulfidbrücken miteinander verknüpft sind. Die A-Kette besteht aus 21 Aminosäuren, die B-Kette aus 30 Aminosäuren. Diese beiden Ketten sind im Vorläufermoelkül, dem Proinsulin, durch ein Peptid, das C-Peptid, miteinander verknüpft. Das C-Peptid im Humanproinsulin besteht aus 35 Aminosäuren. Das C-Peptid wird im Rahmen des Reifungsprozesses des Hormons durch spezifische Proteasen abgespalten und das Proinsulin so in Insulin überführt (Davidson et al., Nature 333, 93-96, 1988). Neben den natürlich vorkommenden C-Peptiden sind in der Literatur eine Vielzahl von Verbindungsmöglichkeiten zwischen A-Kette und B-Kette beschrieben (Yanaihara et al. Diabetes 27, 149-160 (1978), Busse et al, Biochemistry 15, 1649-1657 (1971), Geiger et al., Biochem. Biophys. Res. Com. 55, 60-66 (1973)).

Im Rahmen der Gentechnologie ist es heute möglich, Insulin aus gentechnisch veränderten Mikroorganismen herzustellen. Wird als Mikroorganismus E. coli verwendet, so wird das Produkt häufig als Fusionsprotein exprimiert, d.h. das Produkt wird mit einem bakterieneigenen Protein gekoppelt, z.B. mit der ß-Galaktosidase. Dieses Fusionsprotein fällt in der Zelle aus und ist so vor proteolytischem Abbau geschützt. Nach Zellaufschluß wird der Fusionsproteinanteil chemisch oder enzymatisch abgespalten und die 6 Cysteine des Insulinvorläufers mittels oxidativer Sulfitolyse in ihre S-Sulfonate ($-S-SO_3^-$) überführt. Aus diesem sogenannten Präproinsulin-S-Sulfonat muß in einem folgenden ("Renaturierungs- oder Rekombinations-")Schritt natives Präproinsulin unter Ausbildung der 3 korrekten Disulfidbrücken - d.h. -S-S-Brücken von A6 nach A11, von A7 nach B7 und von A20 nach B19 in den entsprechenden Insulin-Peptidsequenzen - erzeugt werden.

Dieser Schritt erfolgt z.B. nach dem in der EP-B-00 37 255 beschriebenen Verfahren durch Umsetzung des Ausgangs-S-Sulfonats mit einem Mercaptan in einer Menge, die 1 bis 5 SH-Reste pro $SSO_3^-$Rest ergibt, in wäßrigem Medium bei einem pH-Wert von 7 bis 11,5 und einer S-Sulfonatkonzentration von bis zu 10 mg pro ml wäßriges Medium vorzugsweise in Abwesenheit eines Oxidationsmittels.

Dabei sollen Ausbeuten von z.T. über 80 % erhalten werden.

Bei diesem sowie praktisch auch bei allen anderen bekannten Renaturierungs- oder Rekombinations-Verfahren, bei denen Insulin-Vorprodukte mit geöffneten Disulfidbrücken in Produkte mit den entsprechend geschlossenen Disulfidbrücken umgewandelt werden, entstehen - je nach den Reaktionsbedingungen und insbesondere den Konzentrationsverhältnissen - neben den (erwünschten) Renaturierungsprodukten mit korrekten Disulfidbrücken immer auch mehr oder weniger erhebliche Mengen an (unerwünschten) "falschen" Rekombinanten, d.s. Insulinprodukte mit nur teilweise korrekten oder überhaupt keinen korrekten Disulfidbrücken sowie auch mit intermolekularen Disulfidbrücken.

Wenn die nach den bekannten Renaturierungsverfahren erhaltenen Insulin-Vorläuferprodukte ohne Abtrennung der "falschen" Rekombinanten auf Insulin aufgearbeitet werden -was nach bekannten Techniken (chemisch oder enzymatisch) geschehen kann - entsteht aus den "falschen" Rekombinanten kein (natives) Insulin.

Es ist daher zweckmäßig bzw. notwendig, die "falschen" Rekombinanten vor der Aufarbeitung der entsprechenden Renaturierungsprodukte zum Insulin von den Renaturierungsprodukten mit den korrekten Disulfidbrücken abzutrennen. Dies kann z.B. nach bekannten chromatographischen Verfahren geschehen. Nach einem besonders vorteilhaften anderen Verfahren erfolgt die Abtrennung durch Einstellung des Reaktionsgemisches auf pH 4 bis 6 - vorzugsweise in Gegenwart einer geringen Menge einer physiologisch unbedenklichen oberflächenaktiven Substanz wobei die "richtigen" Rekombinanten praktisch vollständig in Lösung bleiben und die "falschen" Rekombinanten ausgefällt werden (vgl. DE-A-35 01 601).

Die abgetrennten "falschen" Rekombinanten werden dann zweckmäßig wieder durch Sulfitolyse in das entsprechende S-Sulfonat überführt, welches einer erneuten Faltung unterworfen wird, wobei es häufig notwendig ist, Nebenprodukte, die bei der Sulfitolyse entstehen, vor der Renaturierung durch Chromatographie zu entfernen. Auf diese Weise lassen sich die "falschen" Rekombinanten wieder weitgehend in "brauchbares" Produkt überführen.

Die Sulfitolyse der "falschen" Rekombinanten mit anschließender Chromatographie und erneuter Faltung bedeutet natürlich einen nicht unerheblichen Aufwand.

In dem Bestreben, diesen Aufwand zu vermeiden oder wenigstens zu vermindern, wurde nun gefunden, daß dies durch Umsetzung der "falschen" Rekombinanten von Insulin-Vorläufern in wäßrigem Medium mit überschüssigem Mercaptan in Gegenwart eines organischen Redox-Systems oder mindestens einer organischen Verbindung, welche unter den Reaktionsbedingungen ein solches organisches Redox-System bildet, gelingt.

Dadurch lassen sich die "falschen" Rekombinanten ohne Sulfitolyse mit hohen Ausbeuten direkt in die

"richtigen" Renaturierungsprodukte bzw. "richtigen" Rekombinanten überführen, was gegenüber dem Stand der Technik einen erheblichen Vorteil darstellt.

Es gibt auch keinerlei Stand der Technik, welcher die Umgehung von Sulfitolyse mit nachfolgender Faltungsstufe lediglich durch Umsetzung mit überschüssigem Mercaptan und den Zusatz eines organischen Redox-Systems in irgendeiner Weise nahegelegt hätte.

Als "falsche" Rekombinanten von Insulin-Vorläufern kommen für das erfindungsgemäße Verfahren vorzugsweise die Produkte in Frage, welche bei der Rekombination von Insulin-Vorläufern mit geöffneten -S-S-Brücken der nachstehenden Formel I als Nebenprodukte entstehen:

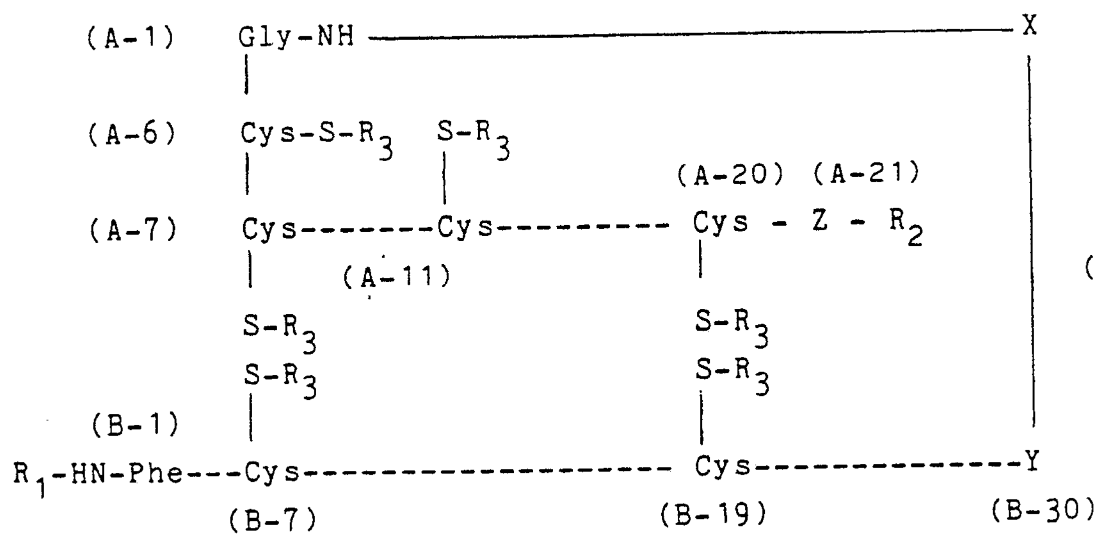

(I)

worin $R_1$ = H,
ein chemisch oder enzymatisch abspaltbarer Aminosäure- oder Peptidrest,
$R_2$ = OH oder ein Aminosäure- oder Peptidrest, vorzugsweise OH
$R_3$ = H oder
eine Cys-S-Schutzgruppe, vorzugsweise die $-SO_3^-$- oder die tert.-Butyl-Gruppe,
X = ein die Insulin A- und B-Kette verbindender Rest, vorzugsweise ein Aminosäure- oder Peptidrest,
Y = Rest einer genetisch kodierbaren Aminosäure, vorzugsweise Thr, Ala, Ser, insbesondere Thr,
Z = Rest einer genetisch kodierbaren Aminosäure, vorzugsweise Asn, Gln, Asp, Glu, Gly, Ser, Thr, Ala oder Met, insbesondere Asn,
A1-A20 und B1-B29 = unmutierte oder durch Austausch einer oder mehrerer Aminosäuren mutierte Peptidsequenzen des Insulins, vorzugsweise die unmutierten Peptidsequenzen des Human-, Schweine-oder Rinderinsulins, insbesondere des Human- oder Schweineinsulins.

Wenn in der Formel I $R_1$ = H, handelt es sich um sich vom Proinsulin ableitende Produkte; Wenn $R_1$ = chemisch oder enzymatisch abspaltbarer Aminosäure- oder Peptidrest, handelt es sich um Produkte, welche sich vom Präproinsulin ableiten.

Chemisch abspaltbare Aminosäurereste sind solche, welche zB. mittels BrCN oder N-Bromsuccinimid abgespalten werden können; dies sind z.B. Methionin (Met) oder Tryptophan (Trp).

Enzymatisch abspaltbare Aminosäurereste sind solche, welche z.B.mittels Trypsin abspaltbar sind (wie z.B. Arg oder Lys).

Chemisch oder enzymatisch abspaltbare Peptidreste sind Peptidreste mit wenigstens 2 Aminosäuren.

Sämtliche für $R_1$ in Frage kommenden Aminosäuren sind bevorzugt aus der Gruppe der natürlichen Aminosäuren, d.s. hauptsächlich Gly, Ala, Ser, Thr, Val, Leu, Ile, Asn, Gln, Cys, Met, Tyr, Phe, Pro, Hyp, Arg, Lys, Hyl, Orn, Cit und His.

$R_2$ ist OH oder - ähnlich $R_1$ - ebenfalls ein Aminosäure-oder Peptidrest, wobei die Bedeutung OH bevorzugt ist. Die Aminosäuren (auch diejenigen, welche den - aus mindestens 2 Aminosäureresten bestehenden - Peptidrest bilden) stammen -wie bei $R_1$ - vorzugsweise aus der Gruppe der natürlichen Aminosäuren.

$R_3$ ist Wasserstoff oder eine Cystein-Schwefel-Schutzgruppe, wobei die $-SO_3^-$ - oder die tert.-Butyl-Gruppe bevorzugte Cystein-Schwefel-Schutzgruppen sind.

X ist ein die Insulin-A- und -B-Kette verbindender Rest, vorzugsweise ein Aminosäure- oder Peptidrest.

Wenn X ein Aminosäurerest ist, ist der Rest von Arg oder Lys bevorzugt; wenn X ein Peptidrest ist, ist der Rest eines natürlich vorkommenden C-Peptids - insbesondere des Human-, Schweine- oder Rinder-Insulin-C-Peptids -bevorzugt.

Genetisch kodierbare Aminosäuren - für Y - sind (jeweils in der L-Form): Gly, Ala, Ser, Thr, Val, Leu, Ile, Asp, Asn, Glu, Gln, Cys, Met, Arg, Lys, His, Tyr, Phe, Trp, Pro.

Bevorzugte genetisch kodierbare Aminosäuren sind Thr, Ala und Ser, insbesondere Thr.

Z kann - wie Y - ebenfalls den Rest einer genetisch kodierbaren Aminosäure bedeuten, wobei hier aber Asn, Gln, Asp, Glu, Gly, Ser, Thr, Ala und Met, insbesondere Asn, bevorzugt sind.

A1 - A20 und B1 - B29 können im Prinzip die unmutierten oder durch Austausch einer oder mehrerer Aminosäuren mutierten Peptidsequenzen aller möglichen Insuline sein; die Mutanten können nach bekannten gentechnologischen Verfahren (site directed mutagenesis) erzeugt werden. Bevorzugt sind jedoch die unmutierten Peptidsequenzen des Human-, Schweine- oder Rinderinsulins, insbesondere des Human- oder Schweineinsulins (die A1 - A20 und B1 - B29-Sequenzen des Human- und Schweineinsulins sind identisch).

Die bei der Rekombination von Insulin-Vorläufern mit geöffneten -S-S-Brücken der Formel I als Nebenprodukte entstehenden "falschen" Rekombinanten werden von den "richtigen" Rekombinanten auf bekannte Weise - vorzugsweise durch Ausfällung bei pH-Werten von 4 bis 6 gemäß dem Verfahren der vorerwähnten DE-A-35 01 641 - und dann direkt oder etwa auch nach vorheriger Gefriertrocknung zum Einsatz für das erfindungsgemäße Verfahren in Wasser bzw. einer wäßrigen Lösung gelöst.

Die Konzentration der "falschen" Rekombinanten in der wäßrigen Ausgangslösung kann in einem weiten Bereich variiert werden; bevorzugte Konzentrationen liegen bei etwa 0,1 bis etwa 100 mg, insbesondere bei etwa 0,1 bis etwa 10 mg/ml, wobei sich die mg-Werte auf die "falschen" Rekombinanten als getrockneten Feststoff beziehen.

Als Mercaptane kommen für die erfindungsgemäße Umsetzung im Prinzip alle möglichen organischen Verbindungen mit SH-Gruppen in Betracht; bevorzugt sind Mercaptoethanol, Thioglycolsäure, Dithioerythrit, Dithioerythritol, Glutathion und Cystein, insbesondere Mercaptoethanol und Cystein. Die Mercaptane können einzeln oder in Mischung eingesetzt werden.

Die einzusetzende Mercaptanmenge kann innerhalb weiter Grenzen schwanken; bevorzugt ist ein Mercaptanüberschuß entsprechend einem Verhältnis von Mercaptan-SH-Gruppen/Cystein-S-Einheiten (in den "falschen" Rekombinanten) von mindestens etwa 5. Nach oben ist dieses Verhältnis praktisch nur durch wirtschaftliche Erwägungen begrenzt. Zweckmäßig ist eine Obergrenze von etwa 100.

Wegen des weiten Schwankungsbereiches des Mercaptanüberschusses braucht in dem eingesetzten "falschen" Rekombinanten die Zahl der Cystein-S-Einheiten nicht ganz exakt bestimmt werden.

Als organische Redox-Systeme kommen bevorzugt Verbindungspaare in Frage, deren eine Komponente eine organische Verbindung mit dem Strukturelement der Formel II

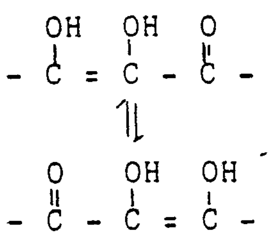

(II)

oder eine aromatische o- oder p-Dihydroxyverbindung und deren andere Komponente eine organische Verbindung mit dem Strukturelement der Formel II in oxidierter Form = Strukturelement der Formel II′

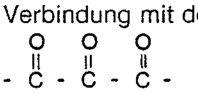

(II′)

oder ein o- oder p-Chinon ist.

Die freien Valenzen des Strukturelementes der Formel II bzw. II′ können durch Wasserstoff oder organische Gruppen wie z.B. $C_1$-$C_4$-Alkylgruppen abgesättigt sein. Das Strukturelement kann aber auch Teil eines Ringes mit vorzugsweise 4,5 oder 6 C-Ring-Atomen sowie gegebenenfalls noch einem oder zwei Heteroatomen wie z.B. O, sein, wobei der Ring seinerseits wieder durch unter den Reaktionsbedingungen inerte Gruppen wie z.B. Alkyl- oder Hydroxyalkylgruppen, substituiert sein kann.

Beispielhafte Verbindungen mit dem Strukturelement der Formel II sind:

Redukton

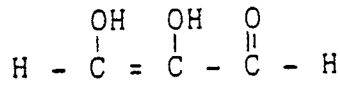

Reduktinsäure

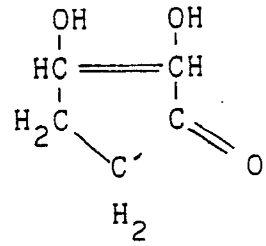

Methylreduktinsäure

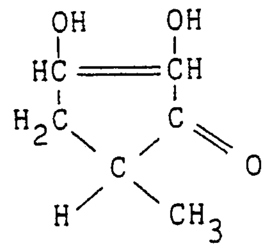

Ascorbinsäure
(Vitamin C)

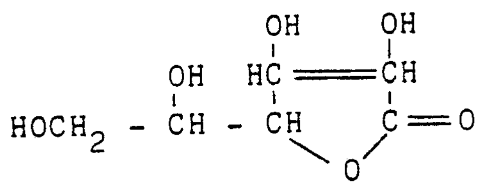

Die Formeln sind hier jeweils nur in der einen tautomeren Form geschrieben.

Sämtliche Verbindungen sind reduzierend. In der oxidierten Form wird aus dem Strukturelement der Formel II dasjenige der Formel II′.

Als aromatische o- und p-Dihydroxyverbindungen kommen im Prinzip alle möglichen aromatischen Verbindungen mit zwei OH-Gruppen in o- oder p-Position in Frage, wobei lediglich noch erforderlich ist, daß die o- bzw. p-Chinon-Bildung aus den o- und p-Dihydroxyverbindungen nicht durch irgendwelche besonderen Substituenten oder dergleichen behindert sein darf. Beispielhafte aromatische o- und p-Dihydroxyverbindungen sind 1,2-Dihydroxybenzol = Brenzkatechin, 1,4-Dihydroxybenzol = Hydrochinon, Methyl-hydrochinon, Naphtho-hydrochinon-1,4, und Anthra-hydrochinon; bei der Oxidation entstehen daraus die entsprechenden Chinone.

Bei der erfindungsgemäßen Umsetzung können nun die jeweiligen organischen Redox-Systeme, bestehend z.B. aus den Verbindungspaaren Ascorbinsäure + Dehydroascorbinsäure, Brenzkatechin + o-Chinon, Hydrochinon + p-Chinon, Naphthohydrochinon + Naphthochinon, etc. in praktisch beliebigem Verhältnis (vorzugsweise in etwa äquimolarem Verhältnis) eingesetzt werden. Es ist aber auch möglich, nur die jeweiligen Einzelkomponenten dieser Verbindungspaare - also z.B. nur Ascorbinsäure oder nur Dehydroascorbinsäure oder nur Hydrochinon usw. - zuzusetzen, weil sich in dem Reaktionsmedium die zu dem Redox-Verbindungspaar gehörige, jeweils andere Komponente (Dehydroascorbinsäure bzw. Ascorbinsäure bzw. p-Chinon usw.) bildet.

Bevorzugte organische Redox-Systeme sind die aus den Verbindungspaaren
Ascorbinsäure + Dehydroascorbinsäure,
Brenzkatechin + o-Chinon und
Hydrochinon + p-Chinon
bestehenden Kombinationen,
und bevorzugte Einzelverbindungen, welche unter den Reaktionsbedingungen ein solches Redox-System bilden, sind die Einzelkomponenten dieser Verbindungspaare.

Ganz besonders bevorzugt sind Ascorbinsäure und/oder Dehydroascorbinsäure.

Die eingesetzte Menge der das organische Redox-System bildende(n) Verbindung(en) kann in weiten

Grenzen variiert werden. Bezogen auf ein Grammäquivalent Mercaptan (= Molekulargewicht des eingesetzten Mercaptans in g/Zahl der SH-Gruppen im Mercaptan-Molekül), kann die Molzahl der das organische Redox-System bildende(n) Verbindung(en) zwischen etwa 1/10 000 und 10 000, vorzugsweise zwischen etwa 1/10 und 10, gewählt werden.

Es ist vorteilhaft, der Reaktionslösung auch Harnstoff zuzusetzen, wobei Konzentrationen entsprechend etwa 0,1 bis 1 M (M = molar), insbesondere von etwa 0,1 - 0,5 M, bevorzugt sind.

Die erfindungsgemäße Umsetzung wird zweckmäßig im alkalischen pH-Bereich, vorzugsweise zwischen etwa 7 und 12, insbesondere zwischen etwa 9,5 und 11, durchgeführt. Zur Aufrechterhaltung des gewünschten pH-Wertes ist der Zusatz einer Puffersubstanz zweckmäßig, wobei Art und Ionenstärke des Puffers einen gewissen Einfluß auf die Faltungsausbeute haben. Es ist vorteilhaft, die Ionenstärke gering zu halten, wobei ein Bereich von etwa 1 mM (mM = millimolar) bis 1 M (M = molar), insbesondere ein solcher von etwa 5 mM bis 50 mM, bevorzugt ist. Als Puffersubstanzen können z.B. Boratpuffer, Carbonatpuffer oder Glycinpuffer verwendet werden, wobei letzterer bevorzugt ist.

Als allgemeiner Bereich der Reaktionstemperatur kann ein solcher zwischen etwa 0 und 45 °C angegeben werden; bevorzugt ist ein Bereich von etwa 4 bis 8 °C.

Die Überschichtung der Renaturierungslösung mit bestimmten Gasen, wie z.B. Sauerstoff, Stickstoff oder Helium hat keinen merklichen Einfluß auf die Renaturierungsausbeute.

Die Umsetzungsdauer liegt im allgemeinen zwischen etwa 2 und 24 Stunden, bevorzugt zwischen etwa 6 und 16 Stunden.

Das Renaturierungsprodukt der erfindungsgemäßen Umsetzung ist - wenn der "falsche" Ausgangs-Rekombinant aus der Rekombination eines Insulin-Vorläufers mit geöffneten S-S-Brücken der vorher erwähnten Formel I stammt - ein Insulin-Vorläufer mit korrekten Disulfidbindungen ("richtiger" Rekombinant) der Formel III

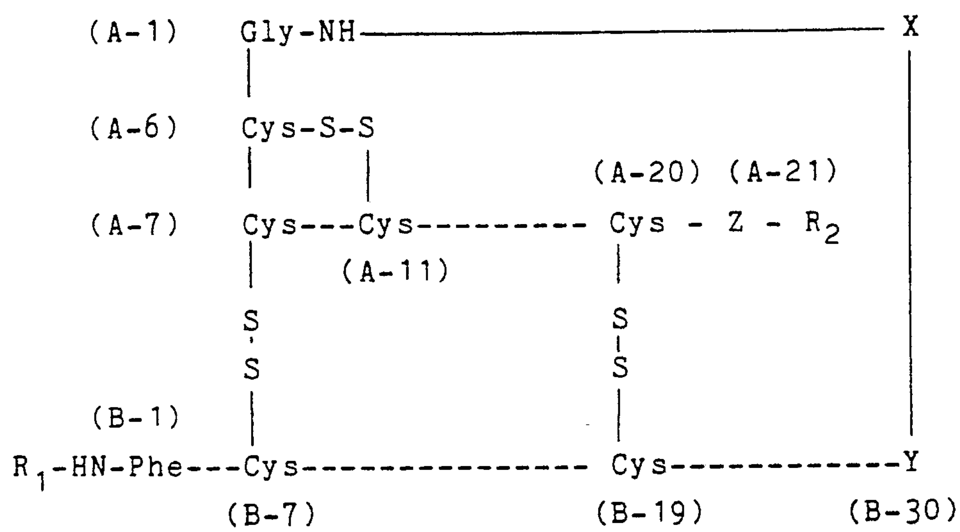

(III)

worin $R_1$, $R_2$, X, Y und Z die gleiche Bedeutung wie in Formel I besitzen.

Nach beendeter Umsetzung (was z.B. durch HPLC festgestellt werden kann) wird in bekannter Weise aufgearbeitet.

Das "richtig" gefaltete Produkt vorzugsweise der Formel III kann dann enzymatisch oder chemisch nach bekannten Techniken in das entsprechende Insulin überführt werden.

Das folgende Beispiel soll die Erfindung näher erläutern. Vor dem (Erfindungs-)Beispiel wird noch die Herstellung des Ausgangsproduktes in beispielhafter Weise beschrieben.

**A) Herstellung des Ausgangsproduktes**

1. Faltung von "Miniproinsulin"

Zum Einsatz kommt "Miniproinsulin-S-$SO_3^-$", d.h. ein Insulin-Vorläufer, bei dem A- und B-Kette des

Insulins über ein Arginin verknüpft sind und die B-kette N-terminal verlängert ist. Das gefriergetrocknete Material (60 %ig) wird mit einer Feststoffkonzentration von 0,5 g/l in 50 mM Glycinpuffer, pH 10,7 gelöst, was einer Vorläuferkonzentration von 0,3 g/l entspricht. Zum Ansatz (100 l) werden 630 ml 1 M Mecaptoethanol und 630 ml 1 M Ascorbinsäure gegeben und anschließend 16 Stunden im Kühlraum bei 8 °C langsam gerührt. Die Faltungsausbeute, bestimmt durch HPLC gegen einen Standard, beträgt 0,228 g/l (76 % der Theorie).

**2. Fällung von Aggregaten ("falsche" Rekombinanten)**

Zu dem Faltungsansatz wird 1 g Polyethylen-polypropylenglykol gegeben und der Gesamtansatz in 5 Ansätze a 20 ml aufgeteilt, mit denen eine pH-Fällungsreihe zwischen pH 5,0 und pH 7,0 in 0,5 pH-Wertschritten erstellt wird. Nach Einstellung des pH-Wertes läßt man 15 Minuten bei Raumtemperatur stehen und zentrifugiert anschließend die Niederschläge ab. Die Überstände werden zur Ermittlung der Fällungsverluste mittels HPLC quantifiziert, die Niederschläge vereinigt und gefriergetrocknet (Auswaage: 15 g Feststoff, Gehalt 40 %ig).
Verlust an richtig gefaltetem Produkt in Abhängigkeit vom pH-Wert:

| | |
|---|---|
| pH 5,0 | 0 % |
| pH 5,5 | 6 % |
| pH 6,0 | 10 % |
| pH 6,5 | 9 % |
| pH 7,0 | 4 % |

Aus dieser Reihe ergibt sich, daß der optimale Fällungs-pH-Wert bei 5,0 liegt.

**B) Erfindungsbeispiel: Renaturierung**

15 g gefriergetrockneter Niederschlag wird in 5 l 4 M Harnstoff aufgenommen. Es werden 13,2 ml Mercaptoethanol (14,35 M) zugegeben (Endkonzentration ca. 35 mM) und 10 Minuten bei Raumtemperatur belassen. Die 5 l Lösung werden zu 25 l 50 mM Glycinpuffer gegeben, es werden 188 ml 1 M Ascorbinsäure zugegeben und der pH-Wert auf 10,7 eingestellt. Der Ansatz wird anschließend 5 Stunden bei 8 °C schwach gerührt. Die Faltungsausbeute beträgt 0,146 g/l (73 % der Theorie). Der Verlauf der Umsetzung wird mittels HPLC verfolgt.
Die Gesamtausbeute der "Miniproinsulin"-Faltung (vgl. A1) läßt sich somit von 76 % der Theorie auf etwa 85 % der Theorie steigern.

**Ansprüche**

1. Verfahren zur Renaturierung "falscher" Rekombinanten von Insulin-Vorläufern, dadurch gekennzeichnet, daß man die "falschen" Rekombinanten in wäßrigem Medium mit überschüssigem Mercaptan in Gegenwart eines organischen Redox-Systems oder mindestens einer organischen Verbindung, welche unter den Reaktionsbedingungen ein solches organisches Redox-System bildet, umsetzt.

2. Verfahren nach Anspruch 1, dadurch gekennzeichnet, daß man als "falsche" Rekombinanten von Insulin-Vorläufern die Produkte verwendet, welche bei der Rekombination von Insulin-Vorläufern mit geöffneten -S-S-Brücken der Formel I als Nebenprodukte entstehen

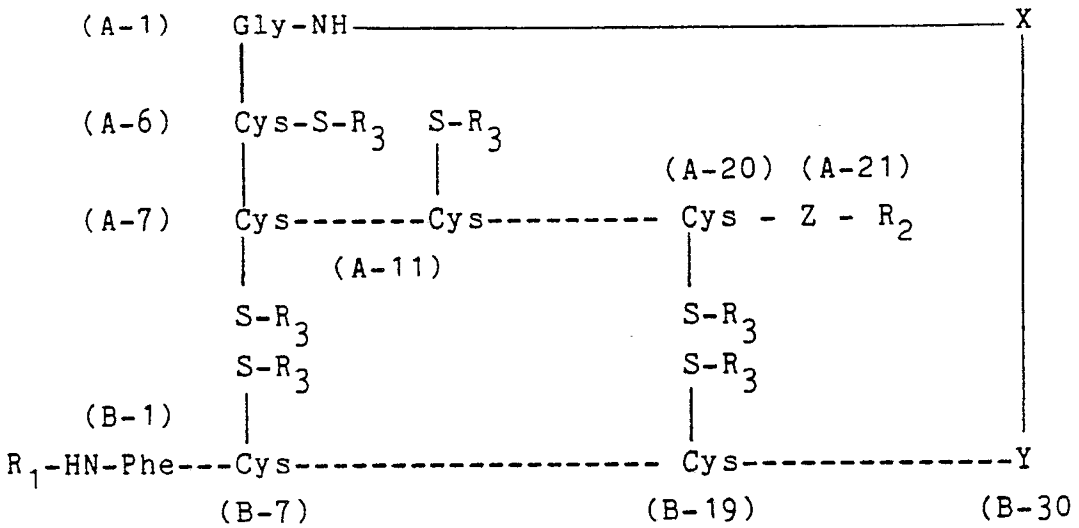

(I)

worin $R_1$ = H,
ein chemisch oder enzymatisch abspaltbarer Aminosäure- oder Peptidrest,
$R_2$ = OH oder ein Aminosäure- oder Peptidrest, vorzugsweise OH
$R_3$ = H oder
eine Cys-S-Schutzgruppe, vorzugsweise die $-SO_3^-$- oder die tert.-Butyl-Gruppe,
X = ein die Insulin A- und B-Kette verbindender Rest, vorzugsweise ein Aminosäure- oder Peptidrest,
Y = Rest einer genetisch kodierbaren Aminosäure, vorzugsweise Thr, Ala, Ser, insbesondere Thr,
Z = Rest einer genetisch kodierbaren Aminosäure, vorzugsweise Asn, Gln, Asp, Glu, Gly, Ser, Thr, Ala oder Met, insbesondere Asn,
A1-A20 und B1-B29 = unmutierte oder durch Austausch einer oder mehrerer Aminosäuren mutierte Peptidsequenzen des Insulins, vorzugsweise die unmutierten Peptidsequenzen des Human-, Schweine- oder Rinderinsulins, insbesondere des Human- oder Schweineinsulins.

3. Verfahren nach Anspruch 1 oder 2, dadurch gekennzeichnet, daß man bei Konzentrationen der "falschen" Rekombinanten von etwa 0,1 bis etwa 100 mg, vorzugsweise von etwa 0,1 bis etwa 10 mg/ml, arbeitet.

4. Verfahren nach einem oder mehreren der Ansprüche 1 bis 3, dadurch gekennzeichnet, daß man als Mercaptan Mercaptoethanol und/oder Cystein verwendet.

5. Verfahren nach einem oder mehreren der Ansprüche 1 bis 4, dadurch gekennzeichnet, daß man mit einem Mercaptan-Überschuß entsprechend einem Verhältnis von Mercaptan-SH-Gruppen/Cystein-S-Einheiten (in den "falschen" Rekombinanten) von mindestens etwa 5, vorzugsweise von etwa 5 bis 100, arbeitet.

6. Verfahren nach einem oder mehreren der Ansprüche 1 bis 5, dadurch gekannzeichnet, daß man als organische Redox-Systeme Verbindungspaare verwendet, deren eine Komponente eine organische Verbindung mit dem Strukturelement der Formel II

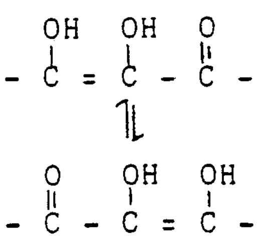

(II)

oder eine aromatische o- oder p-Dihydroxyverbindung, und als andere Komponente eine organische Verbindung mit dem Strukturelement der Formel II in oxidierter Form = Strukturelement der Formel II′

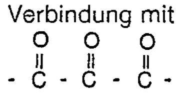

(II′)

oder ein o- oder p-Chinon ist.

7. Verfahren nach einem oder mehreren der Ansprüche 1 bis 5, dadurch gekennzeichnet, daß man als organische Verbindungen, welche unter den Reaktionsbedingungen ein organisches Redox-System bilden,

eine oder mehrere der in Anspruch 6 genannten Einzelkomponenten einsetzt.

8. Verfahren nach einem oder mehreren der Ansprüche 1 bis 7, dadurch gekennzeichnet, daß man als organisches Redox-System die Verbindungspaare
Ascorbinsäure + Dehydroascorbinsäure,
Brenzkatechin + o-Chinon und
Hydrochinon + p-Chinon
und als organische Verbindungen, welche unter den Reaktionsbedingungen ein solches Redox-System bilden können, nur jeweils eine Komponente dieser Verbindungspaare einsetzt.

9. Verfahren nach einem oder mehreren der Ansprüche 1 bis 7, dadurch gekennzeichnet, daß man in Gegenwart von Ascorbinsäure und/oder Dehydroascorbinsäure arbeitet.

10. Verfahren nach einem oder mehreren der Ansprüche 1 bis 9, dadurch gekennzeichnet, daß man das Mercaptan und die das organische Redox-System bildende(n) Verbindung(en) in einem Verhältnis von 1 Grammäquivalent Mercaptan zu 1/10 000 bis 10 000, vorzugsweise 1/10 bis 10 Mol das organische Redox-System bildende(n) Verbindung(en) einsetzt.

11. Verfahren nach einem oder mehreren der Ansprüche 1 bis 10, dadurch gekennzeichnet, daß das wäßrige Reaktionsmedium Harnstoff gelöst enthält, vorzugsweise in etwa 0,1 - 1, insbesondere etwa 0,1-0,5molarer Konzentration.

12. Verfahren nach einem oder mehreren der Ansprüche 1 bis 11, dadurch gekennzeichnet, daß man die Umsetzung bei einem pH-Wert zwischen etwa 7 und 12, vorzugsweise zwischen etwa 9,5 und 11, durchführt.